# EUROPÄISCHE PATENTSCHRIFT

(11) **EP 1 305 276 B1**
(45) Veröffentlichungstag und Bekanntmachung des Hinweises auf die Patenterteilung: **19.05.2004**
(21) Anmeldenummer: 01971794.1
(22) Anmeldetag: 27.07.2001
(51) Int. Cl.: C07C 67/04, C07C 69/54, B01D 1/06, B01D 1/22, C07C 67/54

(54) **VERFAHREN ZUR HERSTELLUNG VON TERT.-C4-C8-ALKYLESTERN DER (METH)ACRYLSÄURE UND DARIN VERWANDTER FALLFILMVERDAMPFER**
METHOD FOR PRODUCING TERTIARY C4-C8-ALKYL ESTERS OF (METH)ACRYL ACID
PROCEDE POUR LA PRODUCTION D'ESTERS DE TERT-ALKYLE C4-C8 DE L'ACIDE (METH)ACRYLIQUE

(30) Priorität: 28.07.2000 DE 10036958
(43) Veröffentlichungstag der Anmeldung: 02.05.2003
(73) Patentinhaber: BASF AKTIENGESELLSCHAFT, 67056 Ludwigshafen (DE)
(72) Erfinder: KROKER, Ruprecht, 67240 Bobenheim-Roxheim (DE); NESTLER, Gerhard, 1070 Wien (AT); SCHMITT, Werner, 67227 Frankenthal (DE); SCHUMM, Winfried, 45886 Gelsenkirchen (DE)
(74) Vertreter: Thalhammer, Wolfgang, Dr.
(86) Internationale Anmeldenummer: PCT/EP2001/008713
(87) Internationale Veröffentlichungsnummer: WO 2002/010110

(56) Entgegenhaltungen:
- DE-B- 1 249 857
- DE-C- 747 514
- US-A- 4 094 734

## Beschreibung

Die vorliegende Erfindung betrifft ein Verfahren zur Herstellung von tert.-C₄-C₈-Alkylestern der (Meth)acrylsäure durch Umsetzung von (Meth)acrylsäure mit einem Olefin der Formel 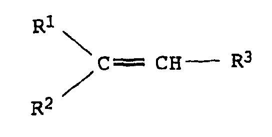 worin
R¹ und R², die gleich oder verschieden sein können, für Methyl oder Ethyl stehen und R³ für H, Methyl oder Ethyl steht, in Gegenwart eines sauren Katalysators in homogener Phase und Gewinnung des Esters aus dem Reaktionsgemisch.

Tert.-Alkylester der (Meth)acrylsäure sind wichtige Ausgangsstoffe zur Herstellung von Polymerisaten, die u. a. als Bestandteil von Anstrichdispersionen, Klebstoffen oder Lackharzen Anwendung finden. Die Herstellung von tert.-Alkylestern erfolgt im Allgemeinen durch säurekatalysierte Addition der entsprechenden Carbonsäuren an Isoolefine (Houben-Weyl, Methoden der Organischen Chemie, Bd. 8, 1952, S. 534; US 3,031,495 und US 3,082,246). Als Katalysatoren verwendet man dabei im Reaktionsgemisch lösliche Säuren, z. B. Mineralsäuren oder Alkyl- bzw. Arylsulfonsäuren (DE-A-12 49 857, US 3,087,962, US 3,088,969) oder unlösliche Katalysatoren wie saure Austauscherharze (US 3,037,052, US 3,031,495, DE-A-31 05 399, EP-A-268 999).

Die Umsetzung der Carbonsäuren mit den Isoolefinen erfolgt in der Regel in herkömmlichen Reaktionsbehältern oder in Säulen (DE-A-11 28 428), wobei die Durchmischung des Reaktionsgemisches durch Rühren oder durch den eingeleiteten Isoolefin-Strom erfolgt. Die Wärmeabfuhr erfolgt in üblicher Weise.

In der Regel treten folgende Schwierigkeiten bei der Herstellung von tert.-Alkylestern auf:
- Oligomerisierung der Isoolefine
- Polymerisation der (Meth)acrylsäure oder der Ester bei thermischer Belastung
- Rückspaltung der tert.-Alkylester unter Wärmeeinwirkung und/oder in Anwesenheit von Spuren an starken Säuren
- Unzureichende Abfuhr der bei der stark exothermen Veresterungsreaktion auftretenden Reaktionswärme, was Rückspaltung des Esters, Oligomerisierung des Isoolefins und Polymerisation der (Meth)acrylverbindungen zur Folge haben kann.

Im Stand der Technik wurden zahlreiche Versuche unternommen, die auftretenden Schwierigkeiten zu verringern oder zu vermeiden. So kann die Oligomerisierungsneigung der Isoolefine durch Erniedrigung der Reaktionstemperatur (US 3,172,905), durch die Anwesenheit von Wasser (US 3,088,969) und durch teilweise Neutralisation des Katalysators (DE-A 31 05 399) verringert werden. Diese Maßnahmen haben jedoch den Nachteil, dass die Reaktionsgeschwindigkeit reduziert wird.

Die Bildung der Isoolefinoligomere kann auch durch die Verwendung von gasförmigen Isoolefinen verringert werden (DE-A-11 35 897). Nachteilig ist dabei jedoch die notwendige Verdampfung des flüssigen Isoolefins und das Handhaben großer Gasmengen.

Aufgrund der oben genannten Schwierigkeiten kommt auch der Gewinnung der tert.-Alkylester aus dem Reaktionsgemisch besondere Bedeutung zu. Häufig wird der saure Katalysator extrahiert und/oder neutralisiert, um die Rückspaltung des tert.-Esters zu verhindern (US 3,172,905; US 3,037,052; DE-A-11 28 428 und DE-A 11 35 897). Diese Methode ist jedoch umweltbelastend und unwirtschaftlich. Außerdem besteht bei Verwendung von Aminen zur Neutralisation des Katalysators die Gefahr, die Polymerisation der (Meth)acrylsäure und/oder des tert.-Esters zu fördern, siehe JP 60 130 546. Die DE-A 12 49 857 schlägt zur Abtrennung des Katalysators (Schwefelsäure) eine schonende Destillation bei einer Temperatur von 30 bis 80°C und einem Druck von 0,1 bis 100 mbar vor. Das erhaltene Sumpfprodukt wird in den Reaktor zurückgeführt und das Kopfprodukt, ein Gemisch aus Ester und Diisoolefin, wird weiter aufgearbeitet. Nachteilig ist jedoch, dass bei dieser Methode das Kopfprodukt trotzdem nicht vollkommen frei von Katalysator ist.

Die starke Polymerisationsneigung von (Meth)acrylverbindungen, vor allem bei höherer Temperatur ist bekannt. Insbesondere bei der destillativen Reinigung sind diese Verbindungen im Allgemeinen Temperaturen ausgesetzt, die leicht eine unerwünschte Polymerisation auslösen können. Dies hat Verschmutzung der Apparaturen, Verstopfen von Leitungen und Pumpen und die Belegung von Kolonnenböden und Wärmeaustauscherflächen zur Folge. Das Reinigen der Anlagen ist ein aufwendiger, teurer und umweltbelastender Vorgang, und die Verfügbarkeit der Anlagen wird dadurch stark reduziert. In diesem Zusammenhang ist von Bedeutung, dass bei der Herstellung von (Meth)acrylsäure, z. B. durch Gasphasenoxidation von Propen oder Acrolein bzw. Isobuten oder Methacrolein an Metalloxidkatalysatoren Essigsäure als Nebenprodukt in einer Menge von bis zu 1 Gew.-% gebildet wird. Essigsäure ist jedoch eine störende Komponente, die vor dem Einsatz der (Meth)acrylsäure abgetrennt werden muß. Aufgrund der geringen Siedepunktsdifferenz und der starken Polymerisationsneigung der (Meth)acrylsäure ist eine Abtrennung der Essigsäure jedoch schwierig und aufwendig (US 3,844,903 und DE-A 21 64 767), so dass die Verwendung von im Wesentlichen Essigsäure-freier (Meth)acrylsäure wirtschaftlich nachteilig ist.

Zur Vermeidung bzw. Verringerung der Polymerisatbildung in (Meth)acrylverbindungen werden in der Regel Polymerisationsinhibitoren beispielsweise Phenothiazin, Hydrochinon, Hydrochinonmonomethylether, p-Nitrosophenol oder t-Butylbrenzkatechin oder Gemische davon, gegebenenfalls unter gleichzeitiger Einwirkung von Luft eingesetzt (DE-A-11 35 897, DE-A-12 49 857, EP-A-0 24 534 und DE-A-29 31 553). Eine vollkommene Verhinderung der Polymerisatbildung ist jedoch nach den bekannten Methoden nicht möglich.

Der vorliegenden Erfindung liegt daher die Aufgabe zugrunde, ein Verfahren zur Herstellung von tert.-C₄-C₈-Alkyl(meth)acrylat zur Verfügung zu stellen, das eine verbesserte Abtrennung des Katalysators ermöglicht, um die Esterrückspaltung möglichst gering zu halten. Eine weitere Aufgabe ist es, ein Verfahren zur Verfügung zu stellen, das den Einsatz Essigsäure-haltiger (Meth)acrylsäure erlaubt. Eine weitere Aufgabe ist es, ein Verfahren zur Verfügung zu stellen, das den gewünschten Ester in hoher Reinheit bei möglichst geringer Polymerisatbildung ergibt. In allen Fällen soll das Verfahren wirtschaftlich und technisch einfach sein.

Überraschenderweise wurde nun gefunden, dass diese Aufgabe gelöst wird, wenn man aus dem durch Umsetzung von (Meth)acrylsäure mit einem Olefin der obigen Formel erhaltenen Reaktionsgemisch den Katalysator durch eine zweistufige Destillation abtrennt und den gewünschten Ester aus den Destillaten gewinnt.

Die vorliegende Erfindung betrifft daher ein Verfahren zur Herstellung von tert.-C₄-C₈-Alkylestern der (Meth)acrylsäure durch Umsetzung von (Meth)acrylsäure mit einem Olefin der Formel 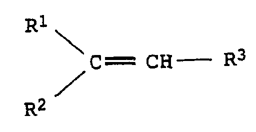 worin
R¹ und R², die gleich oder verschieden sein können, für Methyl oder Ethyl stehen und R³ für H, Methyl oder Ethyl steht, in homogener Phase in Gegenwart eines sauren Katalysators und Gewinnung des tert.-Alkylesters aus dem Reaktionsgemisch, indem man den Katalysator durch eine zweistufige Destillation des Reaktionsgemisches als Rückstand abtrennt und den gewünschten Ester aus den Destillaten gewinnt.

Brauchbare Isoolefine sind z. B. Isobuten, Trimethylethen, 2-Methyl-1-buten oder 2-Ethyl-1-buten, wobei Isobuten besonders bevorzugt ist.

### 1) Veresterung

Die Umsetzung der (Meth)acrylsäure mit dem Olefin der obigen Formel wird in üblicher Weise durchgeführt. Die Art und Weise dieser Umsetzung hat keinen Einfluß auf das erfindungsgemäße Verfahren.

Das Verfahren erfolgt im Allgemeinen in Abwesenheit eines Lösungsmittels und in flüssiger Phase. Als Katalysatoren verwendet man daher solche, die im Reaktionsgemisch zumindest teilweise löslich sind. Brauchbare Katalysatoren sind starke anorganische oder organische Säuren, wie Mineralsäuren beispielsweise Schwefelsäure, Phosphorsäure und Polyphosphorsäure, vorzugsweise Schwefelsäure oder Sulfonsäuren, wie p-Toluol-, Benzol-, Dodecylbenzol-, und Methansulfonsäure.

Die Menge an Katalysator liegt im Allgemeinen im Bereich von etwa 1 bis 10 Gew.-% vorzugsweise 2 bis 5 Gew.-%, bezogen auf (Meth)-acrylsäure.

Das Olefin kann gasförmig und/oder flüssig eingesetzt werden. Die verwendete (Meth)acrylsäure kann Essigsäure bis zu einer Menge von 1 Gew.-% enthalten.

Im Allgemeinen hat die eingesetzte (Meth)acrylsäure folgende Zusammensetzung:

| | | | |
|---|---|---|---|
| (Meth)acrylsäure | 99,0 | bis | 99.9 Gew.-% |
| Essigsäure | 0,05 | bis | 0,5 Gew.-% |
| Propionsäure | 0,02 | bis | 0,4 Gew.-% |
| Wasser | 0,01 | bis | 0,1 Gew.-% |

Die Reaktionspartner können in stöchiometrischen Mengen eingesetzt werden. Im Allgemeinen verwendet man aber einen der Reaktionspartner im Überschuß, vorzugsweise die (Meth)acrylsäure und insbesondere in einem Überschuß von bis zu 50 Mol-%.

Die Reaktionstemperatur liegt im Allgemeinen im Bereich von 20 bis 40°C und die Reaktionszeit beträgt üblicherweise 1 bis 6 Stunden. Der Druck ist nicht kritisch, man kann Unterdruck, Überdruck oder vorzugsweise Umgebungsdruck oder leichten Überdruck (100-300 mbar) verwenden.

Die Umsetzung wird im Allgemeinen auch in Gegenwart eines Inhibitors durchgeführt, der die Polymerisation der (Meth)acrylsäure oder des Esters hemmt. Besonders geeignete Inhibitoren sind Hydrochinon, Hydrochinonmonomethylether, p-Benzochinon, p-Nitroso-phenol, Phenothiazin, N-Oxylverbindungen, z. B. 4-Hydroxy-2,2,6,6-tetramethyl-1-oxyl-piperidin und Methylenblau. Die Inhibitoren kommen im Allgemeinen in Mengen von etwa 200 bis 2000 ppm, bezogen auf das Gewicht der Einsatzstoffe (Meth)acrylsäure und Isoolefin, zur Anwendung.

Das Verfahren kann kontinuierlich oder diskontinuierlich in üblichen Reaktoren durchgeführt werden. Das erhaltene Reaktionsgemisch hat im Falle von Isobuten in der Regel folgende Zusammensetzung:

| | | | |
|---|---|---|---|
| Tert. Butyl(meth)acrylat | 50 | bis | 90 Gew.-% |
| (Meth)acrylsäure | 10 | bis | 30 Gew.-% |
| Tert.-Butanol | 0,1 | bis | 1 Gew.-% |
| Tert.-Butylacetat | 0,1 | bis | 1 Gew.-% |
| Diisobuten | 0,05 | bis | 1 Gew.-% |
| saurer Katalysator | 1 | bis | 5 Gew.-% |
| Polymerisationsinhibitor | 0,05 | bis | 0,2 Gew.-% |

### 2) Katalysatorabtrennung

Das Reaktionsgemisch wird dann in einer zweistufigen Destillation aufgetrennt. Die Destillation erfolgt bei erhöhter Temperatur und bei verringertem Druck. Die Bedingungen richten sich nach dem jeweils gewünschten Produkt. sie werden in der Regel so gewählt, dass die Temperatur im Bereich von etwa 50 bis 150°C liegt. Der Druck wird dann nach dem zur Anwendung kommenden Isoolefin gewählt. Der Druck wird jedoch so eingestellt, dass die Destillation rasch und schonend erfolgt.

Die stufenweise Destillation wird so durchgeführt, dass in der ersten Stufe 40 bis 95 Gew.-%, vorzugsweise 60 bis 90 Gew.-% des gewünschten Esters abdestilliert werden. Das Destillat enthält neben dem Zielester und (Meth)acrylsäure die leichtsiedenden Bestandteile, wie tert.-Butanol, tert.-Butylacetat und Diisobuten (bei Verwendung von Isobuten). Der in der ersten Destillation anfallende Sumpf umfasst im Wesentlichen den restlichen Zielester, (Meth)acrylsäure und hochsiedende Bestandteile, beispielsweise polymere (Meth)acrylverbindungen. 10 bis 100 Gew.-% des Sumpfes werden der zweiten Destillationsstufe zugeführt. Falls nur ein Teil des Sumpfes der zweiten Destillationsstufe zugeführt wird, wird der Rest des Sumpfes in den Reaktor zurückgeführt. In der zweiten Destillationsstufe werden der restliche Zielester und die Hauptmenge an (Meth)acrylsäure (bis zu etwa 90 Gew.-%) abdestilliert. Die Destillate beider Stufen werden zweckmäßigerweise vereinigt oder zusammen kondensiert.

Der Sumpf der zweiten Destillationsstufe umfasst im Wesentlichen den sauren Katalysator, die restliche (Meth)acrylsäure und hochsiedenden Bestandteile, insbesondere polymere (Meth)acrylverbindungen. Bei der zweistufigen Destillation erfolgt somit eine Auftrennung des Reaktionsgemisches in ein Destillat, das im Wesentlichen den Zielester, (Meth)acrylsäure und die erwähnten leichtsiedenden Bestandteile umfasst und in einen Rückstand (Sumpfprodukt), der im Wesentlichen den sauren Katalysator, (Meth)acrylsäure und die erwähnten hochsiedenden Bestandteile umfasst. Das Destillat enthält im Allgemeinen < 20 ppm, insbesondere < 10 ppm, Katalysator.

10 bis 100 Gew.-%, vorzugsweise 30 bis 80 Gew.-%, des zweiten Sumpfproduktes werden ausgeschleust und der Rest wird in den Reaktor zurückgeführt.

Beide Destillationsstufen können in üblichen Vorrichtungen durchgeführt werden. Vorzugsweise verwendet man jedoch Vorrichtungen, die eine rasche Destillation erlauben, beispielsweise Filmverdampfer. Geeignete Filmverdampfer sind dem Fachmann bekannt, siehe beispielsweise Ullmann's Encyclopedia of Industrial Chemistry, 5th Ed., Vol. B3, 2-21 bis 2-24 sowie 3-1 bis 3-25, 1988. Vorzugsweise verwendet man in der ersten Destillationsstufe Fallfilm- oder Fallstromverdampfer und in der zweiten Stufe Dünnschichtverdampfer.

Als besonders bevorzugt hat es sich erwiesen, in der ersten Destillationsstufe einen modifizierten, unten näher beschriebenen Fallfilmverdampfer zu verwenden, bei dem die Verdampferrohre oben geschlossen sind. Jedes einzelne Verdampferrohr ist mit einer separaten Zuführung für das Reaktionsgemisch in den geschlossenen Kopfbereich des Verdampferrohrs ausgestattet. Beispielsweise erfolgt die Zufuhr des Reaktionsgemisches über ein Rohr, das in einer Kammer oberhalb des betreffenden Verdampferrohres mündet. Mit Hilfe dieses modifizierten Fallfilmverdampfers wird die Polymerisatbildung im Kopfteil des Verdampfers verhindert und ein Verlegen bzw. Verstopfen der Verdampferrohre vermieden.

Die Kondensation der Brüden kann in üblicher Weise erfolgen, beispielsweise in Kondensatoren herkömmlicher Bauart. Vorzugsweise verwendet man zwei in Serie geschaltete Kondensatoren, insbesondere Platten- oder Rohrbündelkondensatoren, wobei der zweite Kondensator mit niedrigerer Kühltemperatur betrieben wird. Im Allgemeinen beträgt der Temperaturunterschied etwa 30 bis 50°C, wobei die Kühltemperatur des ersten Kondensators im Bereich von etwa 10 bis 25°C liegt. Auf diese Weise wird eine rasche Destillation und Kondensation ermöglicht und die Polymerisatbildung unterdrückt. Um die Polymerisatbildung noch weiter zu verringern, wird ein im Zielester gelöster Inhibitor in den zweiten Kondensator eingebracht. Als Inhibitor verwendet man vorzugsweise ein Gemisch aus Nitrosophenol und Phenothiazin, das zweckmäßigerweise als Lösung von 0,5 bis 0,7 kg p-Nitrosophenol und 0,5 bis 1,5 kg Phenothiazin in 100 l Zielester eingebracht wird, vorzugsweise im Kopfbereich eines vertikal angeordneten zweiten Kondensators. Die Inhibitorlösung wird in solcher Menge aufgebracht, dass die Inhibitorkonzentration in den vereinigten Kondensaten etwa im Bereich von 100 ppm bis 500 ppm liegt. Das Einbringen des Inhibitors erfolgt in üblicher weise, vorzugsweise wird die Inhibitorlösung eingesprüht. Darüberhinaus hat es sich zur Verringerung der Polymerisatbildung als vorteilhaft erwiesen, die mindestens zehnfache Menge des Destillats (Rohester) in den ersten Kondensator, vorzugsweise im Kopfbereich eines vertikal angeordneten Kondensators, einzubringen und insbesondere einzudüsen.

### 3) Leichtsiederabtrennung

Zur Gewinnung des Zielesters aus den vereinigten Destillaten der Katalysatorabtrennung wird das Destillat in einer herkömmlichen Destillationseinheit aus Verdampfer, Kolonne und Kondensator in ein Kopfprodukt und ein Sumpfprodukt aufgetrennt. Die Destillationstemperatur (Sumpftemperatur) liegt im Allgemeinen im Bereich von 30 bis 60°C. Der Druck wird entsprechend je nach Produkt gewählt.

Das Kopfprodukt enthält die leichtsiedenden Bestandteile, wie tert.-Butylacetat, tert.-Butanol und Diisobuten. Es kann auch noch bis zu 5 Gew.-%, bezogen auf das Kopfprodukt, an Zielester enthalten. Das Sumpfprodukt umfasst im Wesentlichen Zielester und (Meth)acrylsäure.

Als Kolonne kommen übliche Kolonnen mit Schüttungen oder gerichteten Packungen oder mit Glocken-, Ventil- oder Siebböden in Betracht. Vorzugsweise verwendet man jedoch eine Bodenkolonne mit 30 bis 50 Dual-Flow-Böden. Der Zulauf zur Destillationskolonne erfolgt im Allgemeinen im mittleren Bereich.

Gemäß einer Variante setzt man dem Zulauf zur Leichtsiederabtrennung eine geringe Menge (0,01 bis 0,1 Gew.-%, bezogen auf das Destillat der Katalysatorabtrennung) einer basischen Verbindung zu. Als basische Verbindungen kommen beispielsweise Alkali- oder Erdalkaliverbindungen, beispielsweise Natrium-, Kalium- oder Calciumhydroxid, -carbonat oder -hydrogencarbonat, oder primäre, sekundäre und insbesondere tertiäre Amine in Betracht. Die Substituenten am Stickstoff der Amine können C₁-C₂₀-Alkyl-, Aryl- oder C₁-C₁₂-Alkylarylgruppen und ggf. mit Hydroxylgruppen substituiert sein. Auch Polyamine, wie Di- und Triamine sind brauchbar. Vorzugsweise verwendet man Triethylamin, Tributylamin, Tri-2-ethylhexylamin, N,N-Dimethyldodecylamin, N,N-Dimethylanilin, N,N-Dimethylbenzylamin, N,N,N',N'-Tetramethyl-1,3-propandiamin, Triethanolamin, Diethylethanolamin, n-Butyldiethanolamin oder Methyldiisopropanolamin.

Durch die Basenzugabe wird die Rückspaltung des tert.-Alkylesters weitgehend verhindert. Obwohl aus der JP-A-60 130 456 bekannt ist, dass die Zugabe von Aminen die Stabilität von (Meth)acrylverbindungen vermindert, hat sich überraschenderweise gezeigt, dass bei der Basenzugabe keine Polymerisatbildung erfolgt, so dass der Betrieb der Anlage durch Belagbildung an Wärmetauscherflächen, Ablagerungen auf Kolonnenböden und/oder in Leitungen und Behältern nicht beeinträchtigt wird.

Die Kondensation der leichtsiedenden Komponenten erfolgt in üblicher Weise. Vorzugsweise erfolgt die Kondensation in zwei in Serie geschalteten Kondensatoren, beispielsweise Rohrbündelkondensatoren. Die Kühltemperatur des zweiten Kondensators ist dabei um etwa 30 bis 50°C niedriger, der erste Kondensator wird mit einer Kühltemperatur von etwa 10 bis 25°C betrieben. Die Kondensate werden vereinigt und teilweise als Kolonnenrücklauf verwendet. Der Rest des Kondensats wird ausgeschleust. Um die Polymerisatbildung im ersten Kondensator und in der Kolonne zu verhindern, wird eine Lösung eines Inhibitors in Zielester auf den ersten Kondensator aufgebracht. Vorzugsweise verwendet man eine Lösung von 0,5 bis 1,5 kg Phenothiazin und 0,3 bis 0,7 kg p-Nitrosophenol in 100 l Zielester. Die Inhibitorlösung wird in solcher Menge aufgebracht, dass die Inhibitorkonzentration etwa im Bereich von 100 ppm bis 500 ppm liegt.

### 4) Reindestillation

Aus dem Sumpfprodukt der Leichtsiederdestillation wird in einer Destillationseinheit üblicher Bauart (Verdampfer, Kolonne und Kondensator) der Zielester in einer Reinheit von mindestens 99,5% gewonnen. Der anfallende Sumpf enthält mindestens 70 Gew.-% (Meth)acrylsäure und wird wieder in den Reaktor zurückgeführt. Die Destillationstemperatur liegt im Allgemeinen im Bereich von 40 bis 80°C. Der Druck wird entsprechend dem zu destillierenden Ester gewählt.

Die Reindestillation erfolgt unter Verwendung einer herkömmlichen Bodenkolonne, beispielsweise einer Kolonne mit 30 bis 50 Dual-Flow-Böden und Zulauf im mittleren Kolonnenbereich. Der reine Zielester wird über Kopf abgetrennt. Die Kondensation des Zielesters erfolgt vorzugsweise in zwei seriell angeordneten Kondensatoren, insbesondere in Rohrbündelkondensatoren. Die Temperatur des Kühlmittels des zweiten Kondensators ist um etwa 30 bis 50°C niedriger als die des ersten Kondensators, bei dem das Kühlmittel eine Temperatur im Bereich von etwa 10 bis 25°C aufweist. Die vereinigten Kondensate werden teilweise als Kolonnenrücklauf und teilweise zur Stabilisierung des Kolonnenkopfes und des ersten Kondensators verwendet, d. h. zur Vermeidung der Polymerisation im Kolonnenkopf und im ersten Kondensator. Der Rest (ca. 10 Gew.-%) des Zielesters wird ausgeschleust. Zur Vermeidung der Polymerisation im zweiten Kondensator wird eine Lösung eines Inhibitors in Zielester eingebracht. Vorzugsweise verwendet man eine Lösung von 0,5 bis 1 Gew.-% Hydrochinonmonomethylester, wobei die Menge an eingebrachtem Inhibitor so gewählt wird, dass der Inhibitorgehalt der vereinigten Kondensate 10 bis 20 ppm beträgt. Zur Vermeidung der Polymerisation im ersten Kondensator wird ein Teil (etwa die 5 bis 10-fache Menge des ausgeschleusten Zielesters) der vereinigten, stabilisierten Kondensate in das Brüdenrohr eingebracht. Vorzugsweise erfolgt das Einbringen der Kondensate durch Eindüsen in das Brüdenrohr in Gegenrichtung zum Gasstrom, wobei die Düse zweckmäßigerweise im Brüdenrohr im Bereich des Kondensatoreingangs eingebaut ist.

Die Polymerisation in der Kolonne wird zum einen durch den Rücklauf des Kondensates, das 10 bis 20 ppm Inhibitor enthält, unterdrückt. Zum anderen wird außerdem eine Lösung eines weiteren Inhibitors in Zielester auf einen Boden im oberen Kolonnenbereich aufgebracht. Man verwendet vorzugsweise eine Lösung von 0,5 bis 1,5 kg Phenothiazin und 0,3 bis 0,7 kg p-Nitrosophenol in 100 l Zielester, wobei die Menge so bemessen ist, dass der Inhibitorgehalt 50 ppm bis 500 ppm im Verstärkerteil beträgt. Desweiteren wird eine Lösung von 15 bis 30 g Hydrochinonmonomethylether (MEHQ) in 100 l Zielester in die Kolonnenhaube eingebracht. Zweckmäßigerweise erfolgt dies durch Eindüsen über eine zentrisch im Brüdenrohrausgang eingebaute Düse. Gewünschtenfalls kann zur weiteren Stabilisierung in die Destillationseinheit sauerstoffhaltiges Gas, z. B. Luft, eingeblasen werden. Durch diese Maßnahmen ist es möglich, die Polymerisatbildung in den Kondensatoren, den Brüdenrohren und in der Kolonne zu verhindern.

Der erhaltene Ester ist von hoher Reinheit und weist bei Einsatz von Isobuten im Allgemeinen folgende Zusammensetzung auf:

| | | | |
|---|---|---|---|
| tert.-Butyl(meth)acrylat | 99,5 | bis | 99,9 Gew.-% |
| tert.-Butylacetat | 0,001 | bis | 0,01 Gew.-% |
| tert.-Butylpropionat | 0,02 | bis | 0,03 Gew.-% |
| tert.-Butanol | 0,001 | bis | 0,01 Gew.-% |
| (Meth)acrylsäure | 0,005 | bis | 0,02 Gew.-% |
| Hydrochinonmonomethylether | 0,001 | bis | 0,002 Gew.-% |

Das erfindungsgemäße Verfahren hat folgende Vorteile gegenüber dem Stand der Technik:
- Es kann Essigsäure-haltige (Meth)acrylsäure eingesetzt werden. Die schwierige und technisch aufwendige destillative Abtrennung der Essigsäure kann daher unterbleiben und es wird überraschenderweise trotzdem ein tert.-Alkylester hoher Reinheit gewonnen.
- Durch die zweistufige Abtrennung des Zielesters vom Katalysator wird der Restgehalt an Katalysator überraschenderweise erheblich verringert. Die Rückspaltung des Zielesters wird dadurch deutlich vermindert.
- Ein Teil des Katalysator-haltigen Rückstandes wird ausgeschleust und durch frischen Katalysator ersetzt. Die Laufzeit der Produktionsanlage kann dadurch überraschenderweise deutlich verlängert werden.
- Durch die zweistufige Kondensation der Brüden können überraschenderweise eine Polymerisation der (Meth)acrylverbindungen weitgehend verhindert und lange Laufzeiten erreicht werden.
   Dieser Effekt wird noch verstärkt durch die spezielle Stabilisierung von Kolonne und Kondensatoren bei der Leichtsiederabtrennung und der Reindestillation.

Gegenstand der Erfindung ist auch eine Vorrichtung zur Durchführung des erfindungsgemäßen Verfahrens. Es handelt sich dabei um einen Fallfilmverdampfer mit einem zylindrischen Mantel, einer Vielzahl von in dem Mantel angeordneten Verdampfungsrohren, Mitteln zur Zufuhr der zu verdampfenden Flüssigkeit und Mitteln zum Beheizen der Verdampfungsrohre, wobei der Fallfilmverdampfer dadurch gekennzeichnet ist, dass mindestens eines der Verdampfungsrohre einen geschlossenen Kopfbereich aufweist, in den eine Zuleitung für die zu verdampfende Flüssigkeit mündet, wobei die Zuleitung mit den Mitteln zur Zufuhr der zu verdampfenden Flüssigkeit kommuniziert.

In dem zylindrischen Mantel des Fallfilmverdampfers ist eine Vielzahl von Verdampfungsrohren angeordnet, die parallel zur Mantelfläche verlaufen. Im Allgemeinen wird der Mantel im oberen Bereich durch einen meist kuppelförmigen Deckel abgeschlossen, so dass ein Raum ausgebildet wird, der Mittel zur Zufuhr der zu verdampfenden Flüssigkeit und die Zuleitungen zu den Verdampfungsrohren zumindest teilweise aufnehmen kann.

Bei den Mitteln zur Zufuhr der zu verdampfenden Flüssigkeit handelt es sich im Allgemeinen um einen Behälter oder ein Rohr mit größerem Durchmesser als die Zuleitungen. Sowohl der Behälter als auch das Rohr weisen eine Bodenplatte bzw. Abschlußplatte mit Einrichtungen zum Anschluss der Zuleitungen auf. Zweckmäßigerweise handelt es sich dabei um Flansche, Bohrungen, Gewinde etc. in der Bodenplatte, an welchen die Zuleitungen angeschlossen werden können. Die Zuleitungen können auch mit der Bodenplatte verschweißt sein. Die Mittel zur zufuhr der zu verdampfenden Flüssigkeit sind vorzugsweise zumindest teilweise innerhalb des vom kuppelförmigen Deckel am Kopf des Fallfilmverdampfers umschlossenen Raumes angeordnet.

Die Zuleitungen zur Einspeisung der zu verdampfenden Flüssigkeit in die Verdampfungsrohre sind im Allgemeinen als Rohr ausgebildet und verlaufen vom Boden der Mittel zur Zufuhr der zu verdampfenden Flüssigkeit zu den Verdampfungsrohren.

Die Verdampfungsrohre weisen einen abgeschlossenen Kopfbereich auf. Dies kann in unterschiedlicher Weise bewerkstelligt werden, beispielsweise durch eine Platte am oberen Ende der Verdampfungsrohre. Vorzugsweise jedoch ist der geschlossene Kopfbereich als Kammer ausgebildet, die sich zweckmäßigerweise an das Ende des Verdampfungsrohres anschließt. Der Durchmesser der Kammer kann größer als der Durchmesser des jeweiligen Rohres sein. Die Zuleitungen für die zu verdampfende Flüssigkeit ragen, vorzugsweise seitlich, in den abgeschlossenen Kopfbereich.

Darüberhinaus weist der Fallfilmverdampfer Mittel zum Beheizen der Verdampfungsrohre auf, wie sie üblicherweise auf diesem Gebiet zur Anwendung kommen. Beispielsweise können die Verdampfungsrohre von einem flüssigen Wärmeträgermedium umströmt sein. Darüberhinaus weist der Fallfilmverdampfer übliche Mess- und Regeleinrichtungen zur Durchführung des Verfahrens auf.

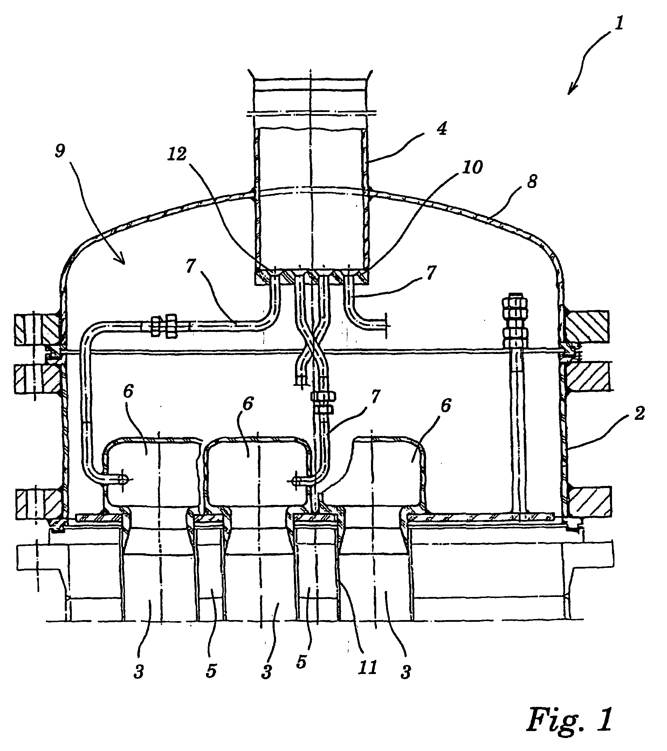

Der Fallfilmverdampfer wird nachfolgend anhand der Figur 1 erläutert.

Fig. 1 zeigt eine schematische Darstellung eines Schnittes durch den Kopfbereich einer bevorzugten Ausführungsform eines erfindungsgemäßen Fallfilmverdampfers.

Der Fallfilmverdampfer 1 wird begrenzt von einem Mantel 2 in zylindrischer Form. Das obere Ende des durch den Mantel gebildeten Zylinders weist einen Deckel 8 auf. Der Mantel umschließt eine Vielzahl von Verdampfungsrohren 3, die im Wesentlichen parallel zur Mantelfläche angeordnet sind. Die Verdampfungsrohre 3 sind so angeordnet, dass ihr Kopfende unterhalb dem Kopfende des Mantels liegt, so dass am Kopf des Fallfilmverdampfers ein vom Mantel 2 und vom Deckel 8 umschlossener Raum 9 gebildet wird. In diesen Raum 9 ragt ein Behälter 4 zur Zufuhr der zu verdampfenden Flüssigkeit.

Die Verdampfungsrohre 3 weisen einen geschlossenen Kopfbereich 6 auf, der als Kammer ausgebildet ist und sich an das Ende der zylindrischen Verdampfungsrohre 3 anschließt. Das in den Raum 9 ragende Ende des Behälter 4 ist mit einem Boden 10 abgeschlossen, welcher Bohrungen zum Anschluss von mehreren Zuleitungen 7 aufweist. Die Zuleitungen 7 führen vom Boden 10 in den geschlossenen Kopfbereich 6 der Verdampfungsrohre 3.

Bei Anwendung des Fallfilmverdampfers wird das Behälter 4 mit der zu verdampfenden Flüssigkeit beaufschlagt. Über die Zuleitungen 7 wird die Flüssigkeit in den geschlossenen Kopfbereich 6 je nach Verdampfungsgeschwindigkeit eingespeist. Die Verdampfung der Flüssigkeit, Kondesation der Brüden, Gewinnung und Austrag des Rückstandes etc. erfolgen dann in üblicher Weise, d. h. die zu verdampfende Flüssigkeit fließt an den Innenflächen der durch ein Wärmeträgermedium 5 beheizten Wände 11 der Verdampfungsrohre 3 nach unten, wobei es zur Verdampfung der leichter flüchtigen Komponenten der Flüssigkeit kommt.

Der erfindungsgemäße Fallfilmverdampfer hat den Vorteil, dass die Verdampfung sehr schonend erfolgt, so dass die Polymerisatbildung beim erfindungsgemäßen Verfahren erheblich reduziert ist.

Die nachfolgenden Beispiele erläutern die Erfindung, ohne sie zu beschränken. Alle Prozentangaben bedeuten Gewichtsprozent.

### Beispiel 1

### Herstellung von tert. Butylacrylat

Als Ausgangsprodukt diente eine Acrylsäure mit im Wesentlichen folgender Zusammensetzung:

| | |
|---|---|
| Acrylsäure | 99,5 % |
| Essigsäure | 0,2 % |
| Propionsäure | 0,03 % |
| Diacrylsäure | 0,1 % |
| Wasser | 0,1 % |
| Hydrochinon-monomethylether (MEHQ) | 0,02 % |

Die gemäß Beispiel 2 der DE 12 49 857 durchgeführte Veresterung von Acrylsäure obiger Zusammensetzung mit Isobuten in Gegenwart von Schwefelsäure und Phenothiazin lieferte ein Veresterungsgemisch, das u. a. folgende Komponenten enthielt:

| | |
|---|---|
| tert. Butylacrylat | 64,0 % |
| tert. Butylacetat | 0,2 % |
| tert. Butanol | 1,6 % |
| Acrylsäure | 24,1 % |
| Diisobuten | 1,2 % |
| Schwefelsäure | 1,0 % |
| tert. Butylschwefelsäure | 5,3 % |
| Phenothiazin | 0,1 % |

Das Veresterungsgemisch wurde einem Röhrenverdampfer (70°C,70 mbar) zugeführt und 50 % des Zulaufs wurden abdestilliert. Der anfallende Rückstand wurde anschließend über einen Dünnschichtverdampfer (Sambay) geleitet (80°C, 60 mbar), wobei 13 % des ursprünglichen Zulaufs als Destillat abgetrennt wurden. Ein Teil (3 %) des Sumpfproduktes der Sambay-Destillation wurde ausgeschleust, der Rest in die Synthese zurückgeführt.
Die bei den Destillationen gebildeten Brüden wurden gemeinsam in zwei in Serie geschalteten Rohrbündelkondensatoren kondensiert, wobei der erste mit Kühlwasser (20°C) und der zweite mit Sole (-20°C) gekühlt wurde. Die anfallenden Kondensate wurden vereinigt. Die Rohre des Sole-Kondensators wurden am Kopf mit einer Lösung (15 l/h) von 0,8 % Phenothiazin und 0,4 % p-Nitrosophenol in tert. Butylacrylat besprüht. Auf den Kopf des ersten Kondensators wurden ca. 16 m³/h rückgeführtes Kondensat aufgesprüht. Entsprechend der Säure-Base-Titration enthielten die vereinigten Kondensate 19,8 % Acrylsäure und < 10 ppm Schwefelsäure.

Die vereinigten Kondensate wurden zur Leichtsiederabtrennung einer Destillationseinheit zugeführt, die aus einem außenliegenden Umlaufverdampfer (Naturumlauf), einer Destillationskolonne mit 40 Dual-Flow-Böden (Zulauf auf Boden 22) und zwei seriell angeordneten Kondensatoren bestand, wobei der erste Kondensator mit Kühlwasser (20°C) und der zweite mit Sole (-20°C) betrieben wurde. Über den Kopf der Kolonne (38°C, 115 mbar) wurden die leichter als tert.-Butylacrylat siedenden Komponenten, hauptsächlich tert.-Butylacetat, Diisobuten und tert. Butanol, abgetrennt und kondensiert (38 l/h), wobei der erste Kondensator mit einer Lösung (15 l/h) von 0,8 % Phenothiazin und 0,4 % p-Nitroso-phenol in tert. Butylacrylat beaufschlagt wurde.

Die vereinigten Leichtsiederkondensate wurden zum Teil ausgeschleust (38 l/h) und zum Teil als Rücklauf (1250 l/h) auf den obersten Boden der Kolonne aufgebracht.

Das anfallende Sumpfprodukt, hauptsächlich aus tert. Butylacrylat und Acrylsäure bestehend, wurde in einer weiteren Destillationseinheit in ein Kopfprodukt, nämlich tert. Butylacrylat, und ein Sumpfprodukt, hauptsächlich Acrylsäure, das in die Synthese zurückgeführt wurde, aufgetrennt (Reindestillation).

Die Destillationseinheit bestand aus einem außenliegenden Rohrbündelverdampfer, einer Kolonne mit 40 Dual-Flow-Böden (Zulauf auf Boden 18) und zwei seriell angeordneten Kondensatoren, wobei der erste mit Kühlwasser (20°C) und der zweite mit (-20°C) Sole betrieben wurde.

Die vereinigten Kondensate wurden teilweise ausgetragen (1200 l/h), teilweise als Rücklauf (ca. 1,1 m³/h) auf den obersten Boden der Kolonne aufgebracht und zum Teil (12,5 m³/h) in das Brüdenrohr des ersten Kondensators in Gegenrichtung zur Gasströmung eingesprüht. Die Kolonne wurde außderdem durch eine Lösung (15 l/h) von 0,8 % Phenothiazin und 0,4 % p-Nitrosophenol in tert. Butylacrylat, die im oberen Kolonnenbereich (Boden 38) zugeführt wurde, stabilisiert.

In den Kolonnenkopf wurde zusätzlich stündlich ein Gemisch aus 300 l Rücklauf und 6 l einer lösung von 1,2 % Hydrochinonmonomethylether (MEHQ) in tert. Butylacrylat über eine Düse, die zentrisch im Eingang in das Brüdenrohr angeordnet war, eingesprüht. Der zweite Kondensator wurde mit einer Lösung (1,7 l/h) von 1,2 % MEHQ in tert. Butylacrylat beaufschlagt.

Das isolierte tert. Butylacrylat enthielt 150 ppm Acrylsäure und hatte eine Reinheit von 99,94 %.

Die in den einzelnen Destillationsschritten auftretenden Abgase, im Wesentlichen Isobuten, wurden vereinigt und über einen Verdichter wieder der Synthese zugeführt.

### Beispiel 2

Die im Beispiel 1 zweistufig beschriebene Katalysatorabtrennung wurde einstufig im Dünnschichtverdampfer bei 80°C und 60 mbar durchgeführt. Das Kondensat enthielt entsprechend der Säure-Base Titration 100 ppm Schwefelsäure.

### Beispiel 3

In 20 ml Ampullen wurde mit verschiedenen Schwefelsäuremengen versetztes isobutenfreies tert. Butylacrylat (5 ml) 2 Stunden bei 60°C gehalten und anschließend wurde gaschromatographisch der Gehalt an Isobuten in der Gasphase bestimmt.

| Schwefelsäuremenge | Isobutengehalt |
|---|---|
| 0 ppm | < 5 ppm |
| 20 ppm | 27 ppm |
| 100 ppm | 180 ppm |
| 320 ppm | 1370 ppm |

Die Versuche machen deutlich, dass die Esterrückspaltung beim erfindungsgemäßen Verfahren aufgrund des geringeren Schwefelsäuregehaltes erheblich geringer ist als bei der einstufigen Katalysatorabtrennung gemäß Stand der Technik.

### Beispiel 4

Es wurde analog dem Beispiel 2 verfahren.

Dem Teil der vereinigten Kondensate der Katalysatorabtrennung, welcher der Leichtsiederabtrennung zugeführt wurde und 10 ppm Schwefelsäure enthielt, wurden kontinuierlich 50 ppm Tributylamin zugegeben. Das bei der Reindestillation anfallende tert. Butylacrylat hatte eine Reinheit von 99,95 % und enthielt weniger als 50 ppm Isobuten. Es konnte innerhalb von 20 Tagen Betriebszeit keine negative Beeinträchtigung (z. B. Polymerisatbildung) der destillativen Aufarbeitung durch die Aminzugabe beobachtet werden.

## Patentansprüche

1. Verfahren zur Herstellung eines tert.-C₄-C₈-Alkyl(meth)acrylats durch Umsetzung von (Meth)acrylsäure mit einem Olefin der Formel 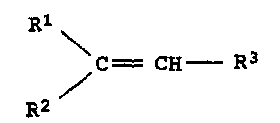 worin
R¹ und R², die gleich oder verschieden sein können, für Methyl oder Ethyl stehen und R³ für H, Methyl oder Ethyl steht, in homogener Phase in Gegenwart eines sauren Katalysators und Gewinnung des tert.-C₄-C₈-Alkyl(meth)acrylats aus dem Reaktionsgemisch, wobei man den Katalysator durch eine zweistufige Destillation des Reaktionsgemisches als Rückstand abtrennt und das tert.-C₄-C₈-Alkyl(meth)acrylat aus den Destillaten gewinnt, wobei man in der ersten Destillationsstufe 40 bis 95 Gew.-% des tert.-C₄-C₈-Alkyl(meth)acrylats abdestilliert und 10 bis 100 Gew.-% des in der ersten Destillationsstufe erhaltenen Rückstandes der zweiten Destillationsstufe zuführt, worin das restliche tert.-C₄-C₈-Alkyl(meth)acrylat und die Hauptmenge an unumgesetzter (Meth)acrylsäure abdestilliert werden.

2. Verfahren nach Anspruch 1, wobei man für die erste Destillationsstufe einen Fallfilmverdampfer mit mehreren Verdampferrohren verwendet, wobei jedes Verdampferrohr einen geschlossenen Kopfbereich aufweist und separat mit dem Reaktionsgemisch beaufschlagt wird.

3. Verfahren nach einem der vorhergehenden Ansprüche, wobei die Kondensation der Destillate mit zwei in Serie geschalteten Kondensatoren erfolgt.

4. Verfahren nach Anspruch 3, wobei eine Lösung eines Polymerisationsinhibitors in tert.-C₄-C₈-Alkyl(meth)acrylat in den zweiten Kondensator eingebracht wird.

5. Verfahren nach einem der vorhergehenden Ansprüche, wobei die vereinigten Destillate der Katalysatorabtrennung einer Destillation in einer Kolonne unterzogen werden, wobei eine Auftrennung in ein die Leichtsieder umfassendes Kopfprodukt und ein das tert.-C₄-C₈-Alkyl(meth)acrylat und (Meth)acrylsäure umfassendes Sumpfprodukt erfolgt.

6. Verfahren nach Anspruch 5, wobei den vereinigten Destillaten der Ratalysatorabtrennung vor der Destillation in der Kolonne 0,01 bis 0,1 Gew.-% einer basischen Verbindung zugesetzt werden.

7. Verfahren nach Anspruch 5 oder 6, wobei die Kondensation des Kopfproduktes in zwei in Serie geschalteten Kondensatoren erfolgt und eine Lösung eines Polymerisationsinhibitors in tert.-C₄-C₈-Alkyl(meth)acrylat in den ersten Kondensator eingebracht wird.

8. Verfahren nach einem der Ansprüche 5 bis 7, wobei das Sumpfprodukt der Leichtsiederabtrennung einer weiteren Destillation in einer Kolonne unterworfen und das tert.-C₄-C₈-Alkyl(meth)acrylat über Kopf abdestilliert wird.

9. Verfahren nach Anspruch 8, wobei das tert.-C₄-C₈-Alkyl(meth)acrylat in zwei in Serie geschalteten Kondensatoren kondensiert und eine Lösung eines Polymerisationsinhibitors in tert.-C₄-C₈-Alkyl(meth)acrylat in den zweiten Kondensator eingebracht wird.

10. Verfahren nach Anspruch 9, wobei die Hauptmenge der vereinigten Destillate in das zum ersten Kondensator führende Brüdenrohr gegen den Gasstrom eingebracht wird.

11. Verfahren nach einem der Ansprüche 8 bis 10, wobei eine Lösung eines Polymerisationsinhibitors in tert.-C₄-C₈-Alkyl(meth)acrylat auf einem Boden im oberen Kolonnenbereich und eine Lösung von Hydrochinonmonomethylether in tert.-C₄-C₈-Alkyl(meth)acrylat in die Kolonnenhaube eingesprüht wird.

12. Verfahren nach einem der vorhergehenden Ansprüche, wobei man als Olefin Isobuten verwendet und tert.-Butyl(meth)acrylat erhält.

13. Fallfilmverdampfer mit einem zylindrischen Mantel (2), einer Vielzahl von in dem Mantel (2) angeordneten Verdampfungsrohren (3), Mitteln (4) zur Zufuhr der zu verdampfenden Flüssigkeit und Mitteln (5) zum Beheizen der Verdampfungsrohre (3), wobei mindestens ein Verdampfungsrohr (3) einen geschlossenen Kopfbereich (6) aufweist, in den eine Zuleitung (7) für die zu verdampfende Flüssigkeit mündet, wobei die Zuleitung (7) mit den Mitteln (4) zur Zufuhr der zu verdampfenden Flüssigkeit kommuniziert, **dadurch gekennzeichnet, dass** die Mittel (4) zur Zufuhr der zu verdampfenden Flüssigkeit als Behälter oder Rohr ausgebildet sind, der bzw. das einen Boden (10) mit Mitteln (12) zum Anschluß der Zuleitung (7) aufweist.

14. Fallfilmverdampfer nach Anspruch 13, **dadurch gekennzeichnet, dass** der geschlossene Kopfbereich (6) als Kammer ausgebildet ist, die an das obere Ende des Verdampfungsrohres (6) anschließt.

15. Fallfilmverdampfer nach Anspruch 13 oder 14, **dadurch gekennzeichnet, dass** der Behälter oder das Rohr (4) in ein von einem Deckel (8) begrenzten Raum (9) am Kopf des Fallfilmverdampfers ragt.

## Claims

1. A process for the preparation of a tert-C₄-C₈-alkyl (meth)acrylate by reacting (meth)acrylic acid with an olefin of the formula 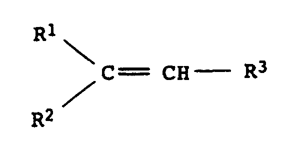 where
R¹ and R², which may be identical or different, are methyl or ethyl and R³ is H, methyl or ethyl, in the homogeneous phase in the presence of an acidic catalyst and isolating the tert-C₄-C₈-alkyl (meth)acrylate from the reaction mixture, wherein the catalyst is separated off as a residue by a two-stage distillation of the reaction mixture and the tert-C₄-C₈-alkyl (meth)acrylate is isolated from the distillates, wherein from 40 to 95% by weight of the tert-C₄-C₈-alkyl (meth)acrylate are distilled off in the first distillation stage and from 10 to 100% by weight of the residue obtained in the first distillation stage are fed to the second distillation stage, the remaining tert-C₄-C₈-alkyl (meth)acrylate and the main amount of unconverted (meth)acrylic acid being distilled off.

2. A process as claimed in claim 1, wherein a falling-film evaporator having a plurality of evaporator tubes is used for the first distillation stage, each evaporator tube having a closed top region and being fed separately with the reaction mixture.

3. A process as claimed in any of the preceding claims, wherein the condensation of the distillates is effected using two condensers connected in series.

4. A process as claimed in claim 3, wherein a solution of a polymerization inhibitor in tert-C₄-C₈-alkyl (meth)acrylate is introduced into the second condenser.

5. A process as claimed in any of the preceding claims, wherein the combined distillates from the catalyst removal are subjected to a distillation in a column, separation into a top product comprising the low boilers and the bottom product comprising the tert-C₄-C₈-alkyl (meth)acrylate and (meth)acrylic acid being effected.

6. A process as claimed in claim 5, wherein from 0.01 to 0.1% by weight of a basic compound is added to the combined distillates from the catalyst removal before the distillation in the column.

7. A process as claimed in claim 5 or 6, wherein the condensation of the top product is effected in two condensers connected in series, and a solution of a polymerization inhibitor in tert-C₄-C₈-alkyl (meth)acrylate is introduced into the first condenser.

8. A process as claimed in any of claims 5 to 7, wherein the bottom product of the low boiler removal is subjected to a further distillation in a column, and the tert-C₄-C₈-alkyl (meth)acrylate is distilled off via the top.

9. A process as claimed in claim 8, wherein the tert-C₄-C₈-alkyl (meth)acrylate is condensed in two condensers connected in series, and a solution of a polymerization inhibitor in tert-C₄-C₈-alkyl (meth)acrylate is introduced into the second condenser.

10. A process as claimed in claim 9, wherein the main amount of the combined distillates is introduced, in a direction opposite to that of the gas stream, into the vapor tube leading to the first condenser.

11. A process as claimed in any of claims 8 to 10, wherein a solution of a polymerization inhibitor in tert-C₄-C₈-alkyl (meth)acrylate is sprayed in on a tray in the upper column region and a solution of hydroquinone monomethyl ether in tert-C₄-C₈-alkyl (meth)acrylate is sprayed into the top of the column.

12. A process as claimed in any of the preceding claims, wherein isobutene is used as the olefin and tert-butyl (meth)acrylate is obtained.

13. A falling-film evaporator comprising a cylindrical jacket (2), a multiplicity of evaporation tubes (3) arranged in the jacket (2), means (4) for feeding in the liquid to be vaporized and means (5) for heating the evaporation tubes (3), wherein at least one evaporation tube (3) has a closed top region (6) into which a feed pipe (7) for the liquid to be vaporized opens, the feed pipe (7) communicating with the means (4) for feeding in the liquid to be vaporized, wherein the means (4) for feeding in the liquid to be vaporized is in the form of a container or tube, which container or which tube has a base (10) with means (12) for connection of the feed pipe (7).

14. A falling-film evaporator as claimed in claim 13, wherein the closed top region (6) is in the form of a chamber which connects to the upper end of the evaporation tube (6).

15. A falling-film evaporator as claimed in claim 13 or 14, wherein the container or the tube (4) projects into a space (9) bounded by a cover (8) and situated at the top of the falling-film evaporator.

## Revendications

1. Procédé de préparation d'un (méth)acrylate de tert-(alkyle en C₄ à C₈) au moyen de la réaction d'acide (méth)acrylique avec une oléfine de formule 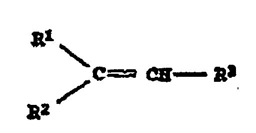 dans laquelle
R¹ et R², qui peuvent être identiques ou différents, représentent un groupe méthyle ou éthyle et R³ représente un atome d'hydrogène, un groupe méthyle ou éthyle en phase homogène en présence d'un catalyseur acide et production du (méth)acrylate de tert-(alkyle en C₄ à C₈) à partir du mélange réactionnel, dans lequel on sépare le catalyseur du mélange réactionnel en tant que résidu au moyen d'une distillation en deux étapes et on produit le (méth)acrylate de tert-(alkyle en C₄ à C₈) à partir des résidus de distillation en distillant, dans la première étape de distillation, 40% à 95% en poids du (méth)acrylate de tert-(alkyle en C₄ à C₈) et en introduisant 10% à 100% en poids du résidu obtenu dans la première étape de distillation dans la deuxième étape de distillation, dans laquelle on distille le (méth)acrylate de tert-(alkyle en C₄ à C₈) restant et la majeure partie de l'acide (méth)acrylique n'ayant pas réagi.

2. Procédé selon la revendication 1, dans lequel on utilise, pour la première étape de distillation, un évaporateur à film descendant équipé de plusieurs tubes d'évaporation, chaque tube d'évaporation présentant une zone de tête fermée et étant alimenté séparément en mélange réactionnel.

3. Procédé selon l'une quelconque des revendications précédentes, dans lequel on réalise la condensation des produits de distillation à l'aide de deux condenseurs montés en série.

4. Procédé selon la revendication 3, dans lequel on introduit une solution d'un inhibiteur de polymérisation dans le (méth)acrylate de tert-(alkyle en C₄ à C₈) dans le deuxième condenseur.

5. Procédé selon l'une quelconque des revendications précédentes, dans lequel on soumet les produits de distillation réunis issus de la séparation du catalyseur à une distillation dans une colonne en réalisant une séparation en un produit de tête de colonne contenant les produits à bas point d'ébullition et en un produit de fond contenant le (méth)acrylate de tert-(alkyle en C₄ à C₈) et l'acide méthacrylique.

6. Procédé selon la revendication 5, dans lequel on ajoute, avant la distillation dans la colonne, 0,01% à 0,1% en poids d'un composé basique aux produits de distillation réunis issus de la séparation du catalyseur.

7. Procédé selon la revendication 5 ou 6, dans lequel on réalise la condensation du produit de tête dans deux condenseurs montés en série et on introduit une solution d'un inhibiteur de polymérisation dans le (méth)acrylate de tert-(alkyle en C₄ à C₈) dans le premier condenseur.

8. Procédé selon l'une quelconque des revendications 5 à 7, dans lequel on soumet le produit du fond issu de la séparation des produits à bas point d'ébullition à une distillation supplémentaire dans une colonne et on distille le (méth)acrylate de tert-(alkyle en C₄ à C₈) en tête.

9. Procédé selon la revendication 8, dans lequel on condense le (méth)acrylate de tert-(alkyle en C₄ à C₈) dans deux condenseurs montés en série et on introduit une solution d'un inhibiteur de polymérisation dans le (méth)acrylate de tert-(alkyle en C₄ à C₈) dans le deuxième condenseur.

10. Procédé selon la revendication 9, dans lequel on introduit, à contre-courant du gaz, la majeure partie des produits de distillation réunis dans le tube de vapeurs allant vers le premier condenseur.

11. Procédé selon l'une quelconque des revendications 8 à 10, dans lequel on pulvérise une solution d'un inhibiteur de polymérisation dans le (méth)acrylate de tert-(alkyle en C₄ à C₈) sur un plateau dans la partie supérieure de la colonne et une solution d'éther monométhylique d'hydroquinone dans le (méth)acrylate de tert-(alkyle en C₄ à C₈) dans la calotte de la colonne.

12. Procédé selon l'une quelconque des revendications précédentes, dans lequel on utilise de l'isobutène en tant qu'oléfine et dans lequel on obtient du (méth)acrylate de tert-butyle.

13. Evaporateur à film descendant présentant une enveloppe cylindrique (2), une pluralité de tubes d'évaporation (3) disposés dans l'enveloppe (2), des moyens (4) pour l'introduction du liquide à évaporer et des moyens (5) pour le chauffage des tubes d'évaporation (3), dans lequel au moins un tube d'évaporation (3) présente une zone de tête fermée (6) dans laquelle débouche une conduite (7) pour le liquide à évaporer, la conduite (7) communiquant avec les moyens (4) pour l'introduction du liquide à évaporer, **caractérisé en ce que** les moyens (4) pour l'introduction du liquide à évaporer sont réalisés sous la forme d'un réservoir ou d'un tube qui présente un fond (10) comprenant des moyens (12) pour le raccordement de la conduite d'alimentation (7).

14. Evaporateur à film descendant selon la revendication 13, **caractérisé en ce que** la zone de tête fermée (6) est réalisée sous la forme de chambre raccordée à l'extrémité supérieure du tube d'évaporation (3).

15. Evaporateur à film descendant selon la revendication 13 ou 14, **caractérisé en ce que** le réservoir ou le tube (4) se prolonge dans un espace (9) limité par un couvercle (8) au niveau de la tête de l'évaporateur à film descendant.
